# EUROPEAN PATENT SPECIFICATION

(11) **EP 4 085 894 B1**
(45) Date of publication and mention of the grant of the patent: **22.07.2026**
(21) Application number: 22171907.3
(22) Date of filing: 05.05.2022
(51) Int. Cl.: A61K 8/34, A61K 8/64, A61K 8/66, A61K 8/67, A61K 8/9794, A61Q 19/00

(54) **COMPOSITION FOR EYE CONTOUR TO PREVENT, REDUCE AND/OR ELIMINATE SKIN IMPERFECTIONS**
ZUSAMMENSETZUNG FÜR DIE AUGENKONTUR, UM HAUTUNEBENHEITEN ZU VERHINDERN, ZU REDUZIEREN UND/ODER ZU BESEITIGEN
COMPOSITION POUR LE CONTOUR DES YEUX POUR PRÉVENIR, RÉDUIRE ET/OU ÉLIMINER LES IMPERFECTIONS DE LA PEAU

(30) Priority: 07.05.2021 IT 202100011738
(43) Date of publication of application: 09.11.2022
(73) Proprietor: Iromed Group S.r.l., 00144 Roma (RM) (IT)
(72) Inventor: CAMPIONE, Elena, Roma RM (IT); COSIO, Terenzio, Torino TO (IT)
(74) Representative: Di Giovine, Paolo

(56) References cited:
- CN-A- 110 538 081
- CN-A- 111 528 421
- CN-A- 112 472 632
- CN-A- 112 618 405
- "Analysis of Cosmetic Products", 1 January 2007, ELSEVIER B.V., article AMPARO SALVADOR ET AL: "Chapter 8.6 Vitamins", pages: 364 - 379, XP055440295

## Description

### FIELD OF THE INVENTION

The present invention refers, in general, to a composition for topical use to be applied preferably in the periocular area in order to prevent, reduce and/or eliminate skin blemishes in the eye contour area. Said composition is used mainly in the cosmetic field, but is also applied in the prevention and/or treatment of capillary fragility and/or periocular edema.

### STATE OF THE ART

The eyelid region is a particularly sensitive and delicate area of the face, much more susceptible to external aggression and premature ageing than the other areas of the face. This sensitivity and susceptibility to aggression is due mainly to the characteristics of the skin in this area. More specifically, this region has thinner skin, fewer sebaceous and sweat glands, a much smaller quantity of subcutaneous adipose tissue, collagen and elastic fibres, with a reduced and slower blood and lymphatic circulation.

For these reasons, dark circles and bags are one of the most widespread and frequent skin blemishes. Their appearance can depend on many factors: incorrect diet, genetic predisposition or allergies. Dark circles are caused by excessive dilation of the capillaries below the eyelid skin, with consequent slowing down of the circulation and the deposit of iron ions, deriving from degradation of the red blood cells, which are responsible for the brownish colour. Periocular edema derives from alteration of the capillary membrane but also from imbibition of the extracellular matrix. It is therefore important to act at several levels, both at cutaneous level and at vascular level, for the treatment of these pathologies and their various manifestations.

### SUMMARY OF THE INVENTION

The technical problem posed and solved by the present invention is that of providing compositions for reducing and treating blemishes in the periocular area, a particularly sensitive and delicate area for many reasons, such as the skin being thinner, a lower number of sebaceous and sweat glands, etc.

This problem is solved by a composition comprising Vitamin K, Bromelain, Resveratrol,

Acetylhexapeptide-8 and mullein extract *(Verbascum thapsus extract).* CN110538081 discloses compositions used in skin care product for accelerating ocular melanin metabolism, reducing dullness, wrinkles and eye bags, eliminating dark circle, relieving eye fatigue, moisturizing and firming eye skin, comprising resveratrol.

Advantageously, the authors of the present invention have discovered that the combination of the active substances present in the composition in question produces an enhanced action in preventing, reducing and/or eliminating blemishes of the periocular skin. For example, the association of Vitamin K, Resveratrol and mullein extract produces an increase in the depigmenting effect since the three active substances act at different levels: the Vitamin K reduces the depigmentation induced by accumulation of the haemoglobin degradation products, the Resveratrol suppresses the melanogenesis, and the iridoids not only give the skin radiance in the area of use, but also screen the cells from the action of the ultraviolet rays.

Therefore, the present invention concerns:
- a composition comprising Vitamin K, Bromelain, Resveratrol, Acetylhexapeptide-8 and mullein extract, according to claim 1;
- a cosmetic product comprising or consisting of the composition comprising Vitamin K, Bromelain, Resveratrol, Acetylhexapeptide-8 and mullein extract according to any one of the embodiments described here;
- use of the composition and/or of the cosmetic product comprising the composition according to any one of the embodiments described here to prevent, reduce and/or eliminate blemishes of the periocular skin.

Preferred characteristics of the present invention are the subject of the dependent claims.

Other advantages, characteristics and methods of use of the present invention will be evident from the following detailed description of some embodiments, presented as non-limiting examples.

### BRIEF DESCRIPTION OF THE FIGURES

The present invention and the following detailed description of the preferred embodiments can be better understood with reference to the following figures:

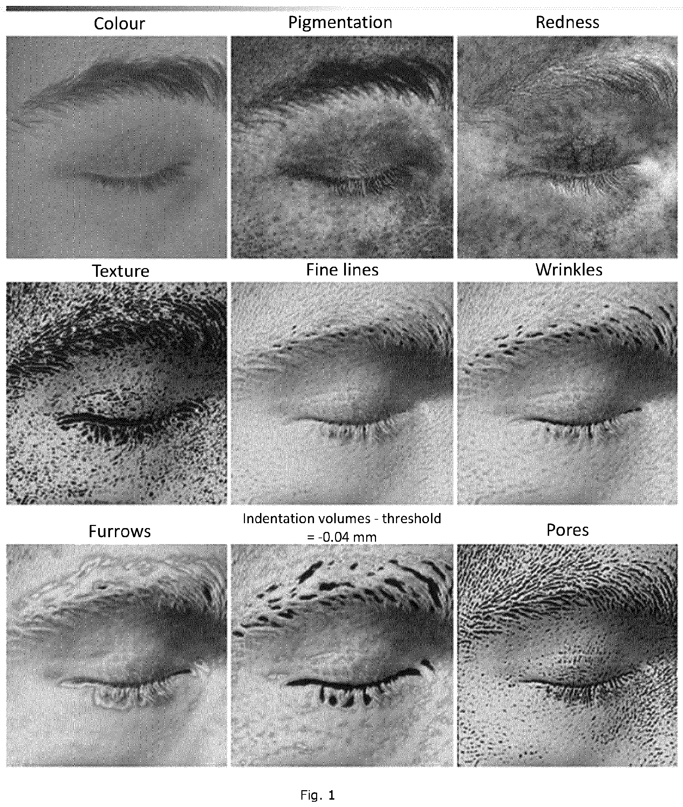

Figure 1. Subject 1, baseline - The image shows the results relative to the 9 parameters specified in Example 2, obtained by means of *in vivo* clinical evaluation on a human (subject 1), at time T0, identified as baseline, namely before the beginning of the treatment. The skin colour corresponds to the one that can be seen by the operator in the macroscopic photo; the pigment highlights the chromophores and specifically the haemosiderin and the melanin at skin level; the redness highlights the surface capillarization; the texture highlights the skin irregularities as a variation of the skin trophism; the fine lines, wrinkles, volumes and furrows fall within the same colour spectrum identifying the skin depressions and the depth/height variations relative to the cutaneous plane; the pores represent the quantity and quality of the hair follicles.

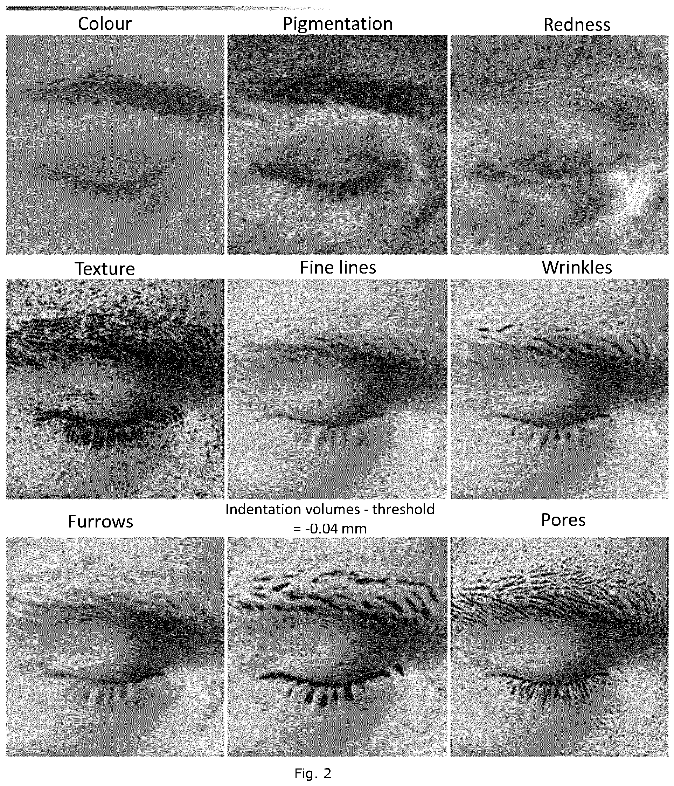

Figure 2. Subject 1, after 21 days of application of a composition according to an embodiment of the invention, comprising Resveratrol (0.1%); Acetylhexapeptide-8 (0.3%); Vitamin K (0.01%); Bromelain (0.1%); mullein extract (1%): after 21 days of application all 9 parameters examined have a positive variation. The colour parameter is attenuated with respect to the basal parameter; the pigmentation parameter is reduced, underlining the topical effect of reduction of the melanin and the haemosiderin; the redness parameter is reduced following the vascular protective effect exercised by the Vitamin K and Resveratrol; the texture, fine lines, furrows and wrinkles parameters are reduced, underlining the synergic action of the Resveratrol (0.1%); Acetylhexapeptide-8 (0.3%); Vitamin K (0.01%); Bromelain (0.1%); mullein extract (1%). Furthermore, a considerable improvement is also noted in the pores parameter at the level of the medial epicanthus and the lower peripalpebral area.

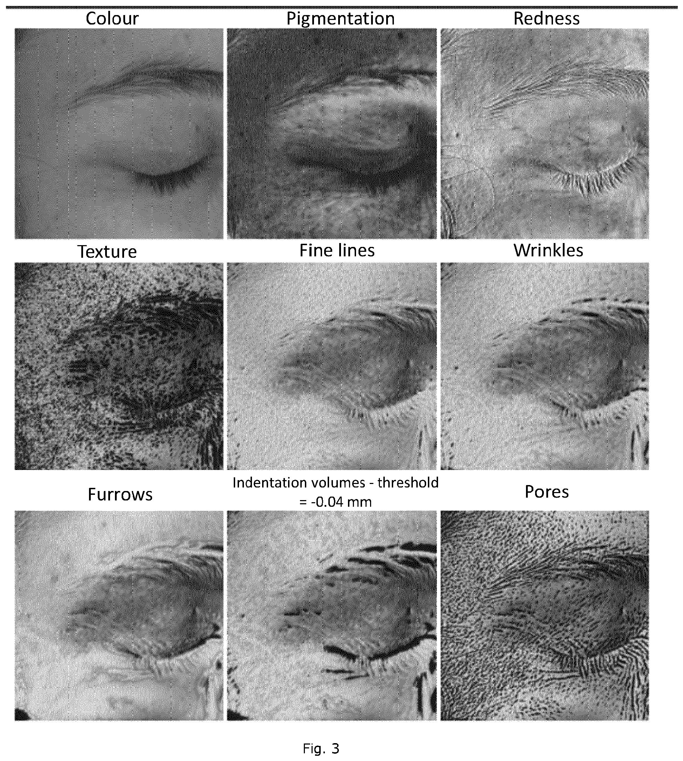

**Figure 3****.** Subject 2, baseline - The image shows the results relative to the 9 parameters specified in Example 2, obtained by means of *in vivo* clinical evaluation on a human (subject 2), at time T0, identified as baseline, namely before the beginning of the treatment. The skin colour corresponds to the one that can be seen by the operator in the macroscopic photo; the pigment highlights the chromophores and specifically the haemosiderin and the melanin at skin level; the redness highlights the surface capillarization; the texture highlights the skin irregularities as a variation of the skin trophism; the fine lines, wrinkles, volumes and furrows fall within the same colour spectrum identifying the skin depressions and the depth/height variations relative to the cutaneous plane; the pores represent the quantity and quality of the hair follicles.

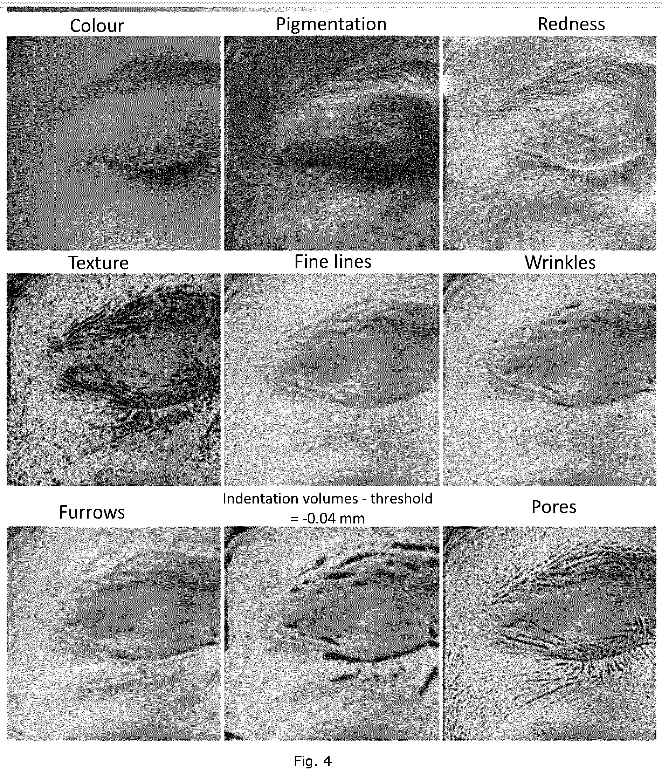

**Figure 4****.** Subject 2, after 21 days of application of a composition comprising base cream/carrier + Acetylhexapeptide-8 (0.3%): after 21 days of application the fine lines, wrinkles, pores redness and skin colour parameters have a positive variation. The colour parameter is attenuated with respect to the basal parameter; the redness parameter is reduced; the fine lines and wrinkles parameters are reduced. Furthermore, a considerable improvement is also noted in the pores parameter at the level of the lower and upper peripalpebral area, and the lateral epicanthus.

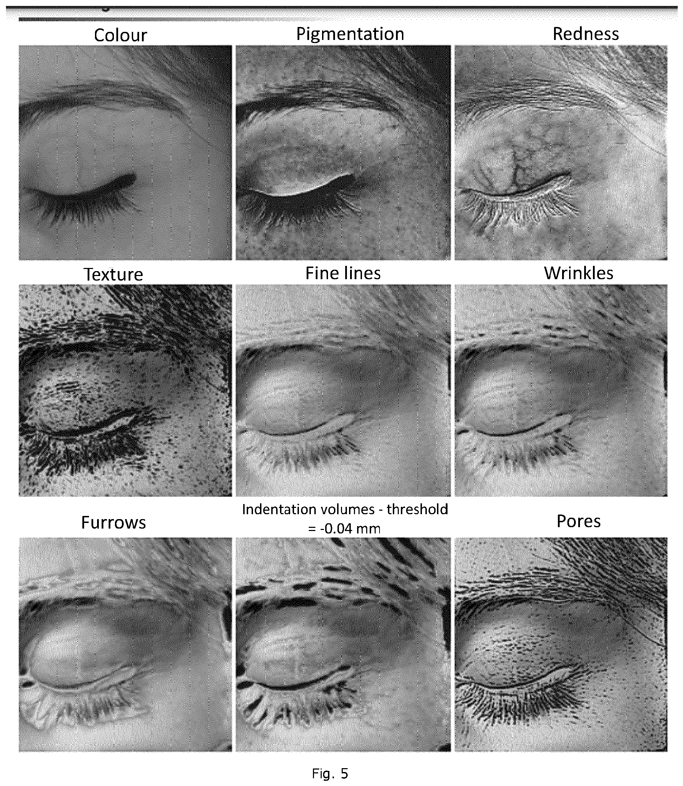

**Figure 5****.** Subject 3, baseline - The image shows the results relative to the 9 parameters specified in Example 2, obtained by means of *in vivo* clinical evaluation on a human (subject 3), at time T0, identified as baseline, namely before the beginning of the treatment. The skin colour corresponds to the one that can be seen by the operator in the macroscopic photo; the pigment highlights the chromophores and specifically the haemosiderin and the melanin at skin level; the redness highlights the surface capillarization; the texture highlights the skin irregularities as a variation of the skin trophism; the fine lines, wrinkles, volumes and furrows fall within the same colour spectrum identifying the skin depressions and the depth/height variations relative to the cutaneous plane; the pores represent the quantity and quality of the hair follicles.

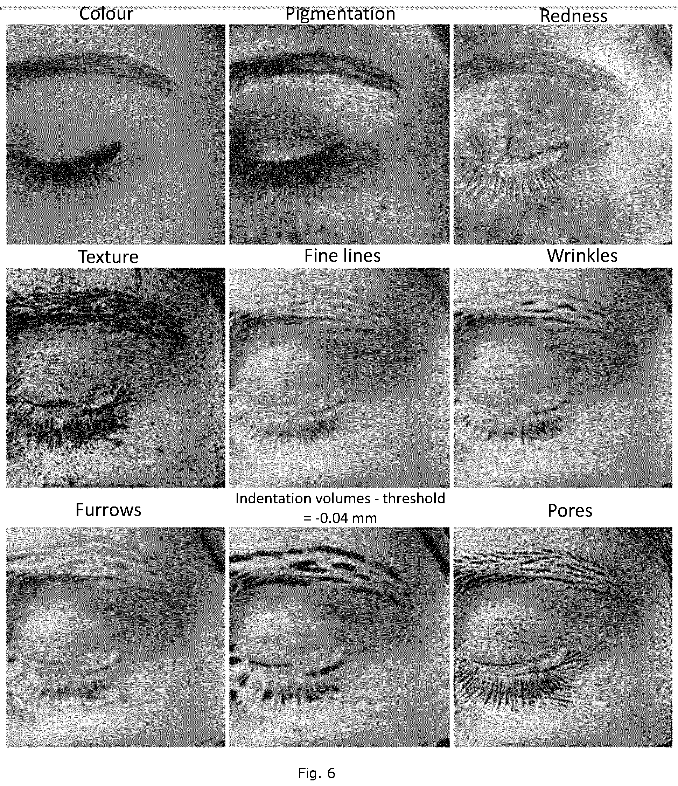

**Figure 6****.** Subject 3, after 21 days of application of a composition comprising base cream/carrier + Vitamin K (0.01%): after 21 days of application the redness parameter has a positive variation.

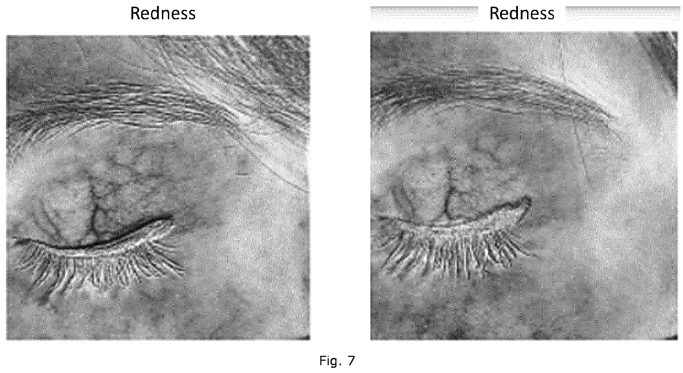

**Figure 7****.** The images show a focus on the "redness" parameter as determined for Subject 3 after 21 days of treatment with a composition comprising base cream/carrier + Vitamin K (0.01%): after 21 days of application a reduction in the redness at the level of the upper eyelid can be noted, identifiable as a reduction in the diameter of the surface capillaries and in the redness at the level of the external epicanthus.

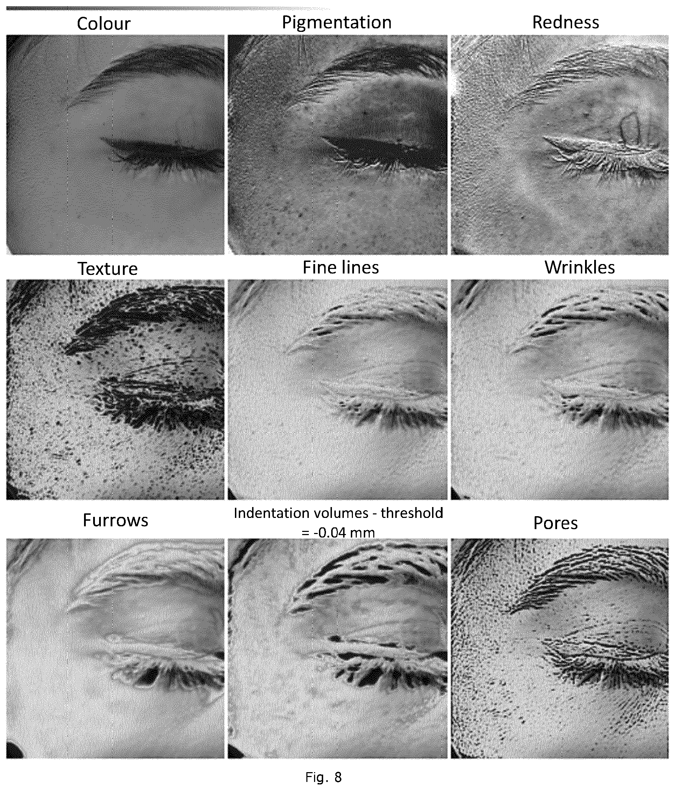

**Figure 8****.** Subject 4, baseline - The image shows the results relative to the 9 parameters specified in Example 2, obtained by means of *in vivo* clinical evaluation on a human (subject 4), at time T0, identified as baseline, namely before the beginning of the treatment. The skin colour corresponds to the one that can be seen by the operator in the macroscopic photo; the pigment highlights the chromophores and specifically the haemosiderin and the melanin at skin level; the redness highlights the surface capillarization; the texture highlights the skin irregularities as a variation of the skin trophism; the fine lines, wrinkles, volumes and furrows fall within the same colour spectrum identifying the skin depressions and the depth/height variations relative to the cutaneous plane; the pores represent the quantity and quality of the hair follicles.

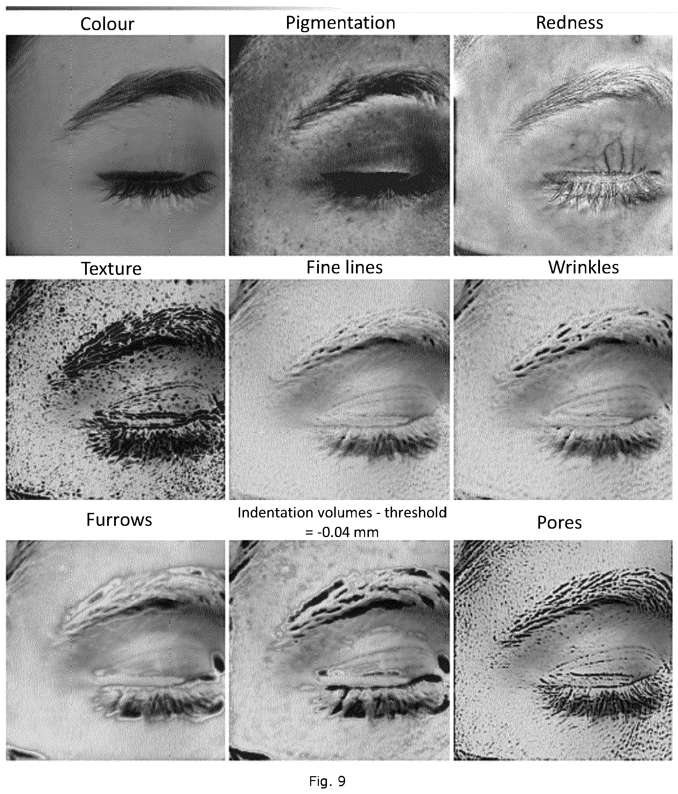

**Figure 9****.** Subject 4, after 21 days of application of a composition comprising base cream/carrier + mullein extract (1%): after 21 days of application the pigmentation and pores parameters show a positive variation. The pigment parameter is attenuated with respect to the basal parameter; the pores parameter is reduced in terms of quantity and quality.

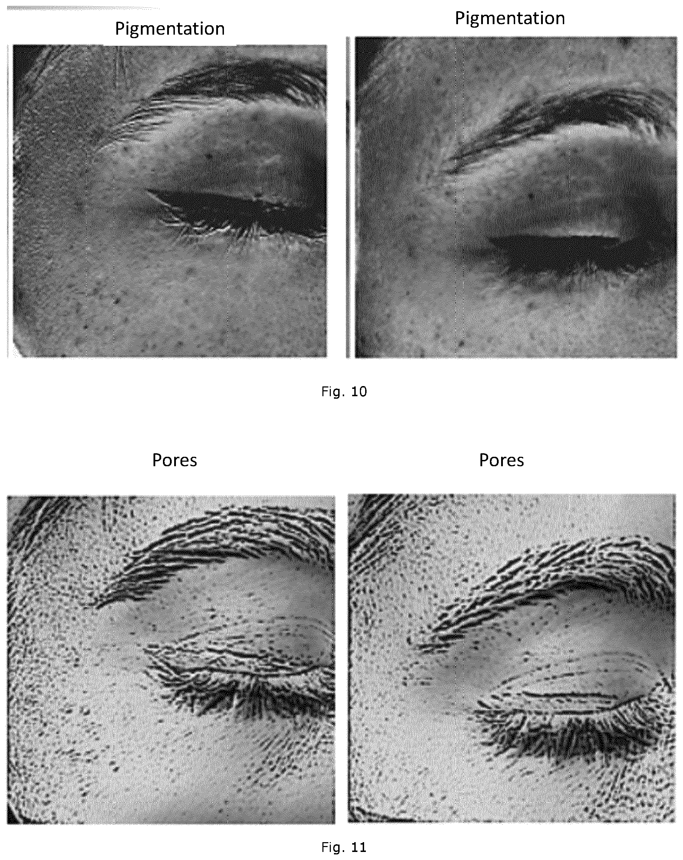

**Figure 10****.** The images show a focus on the "pigmentation" parameter as determined for Subject 4 after 21 days of treatment with a composition comprising base cream/carrier + mullein extract (1%): after topical application for 21 days, the periocular pigmentation, in particular in the lower palpebral portion, shows a reduction.
**Figure 11****.** The images show a focus on the "pores" parameter as determined for Subject 4 after 21 days of treatment with a composition comprising base cream/carrier + mullein extract (1%): there is a reduction in the dimension of the pores at the level of the lateral epicanthus and the median portion of the upper eyelid after 21 days of topical application.

### DETAILED DISCLOSURE OF THE INVENTION

The present invention refers to a composition comprising the following active substances: Vitamin K, Bromelain, Resveratrol, Acetylhexapeptide-8 and mullein extract.

By Vitamin K, the structure of which is the following 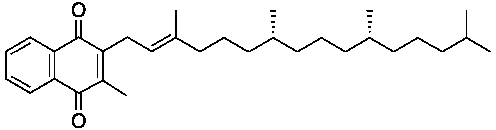 we mean a group of liposoluble vitamers which, at metabolic level, in the biologically active form, guarantee the correct functionality of some specific proteins involved in the calcium bond in bones and in other tissues, and in blood coagulation. This vitamin is useful for preventing dark circles because it is involved in the blood coagulation processes. The microcirculation of the eye contour is characterized by capillaries and small fine blood vessels. It has been shown that Vitamin K acts as an anti-inflammatory agent, suppressing dysregulation of the nuclear factor κB (NF-κB) and exerts a protective effect against oxidative stress, blocking the generation of oxygen reactive species. The clinical evidence available indicates that a high level of Vitamin K can play a protective role in the inflammatory processes associated with the onset and progression of senescence. In the specific case of the periocular microcirculation, Vitamin K acts both by reducing the cutaneous oxidative stress and by strengthening the capillary wall against damage due to ageing. According to an aspect of the present invention, Vitamin K is present in the composition in a concentration between 0.001 % and 0.1% by weight with respect to the total weight of the composition, preferably equal to 0.01%.

Bromelain is a proteolytic enzyme present in the juice, flesh and stem of the pineapple, in which it is found in the most concentrated form and from which, according to a preferred embodiment of the invention in question, it is obtained. Bromelain has been used for years in inflammatory processes due to sports injuries, rheumatoid arthritis, phlebitis and cellulitis. In addition to this anti-inflammatory action, Bromelain has an anti-edemigenous activity (it reduces swelling after a trauma, attenuates water retention) and a platelet anti-aggregant activity (obstructs the formation of thrombi in the blood vessels and is useful in the presence of varicose veins). Its use in the periocular area favours the disappearance of bags under the eyes and has an anti-edemigenous action. Furthermore, by preventing the local accumulation of haemoglobin degradation compounds, it reduces the pigmentation typical of dark circles. According to an aspect of the present invention, Bromelain is present in a concentration between 0.01% and 1% by weight with respect to the total weight of the composition, preferably equal to 0.1%.

Resveratrol, an antioxidant polyphenol of red wine, has a number of unique anti-age properties and its structure is the following:

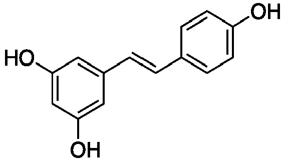

The biological activities of Resveratrol include antioxidant and anti-inflammatory properties, protective activities for the blood vessels, and it is able to stimulate a series of processes involved in regulation of the cell cycle and in the repair of DNA. Its antioxidant capacity contributes to protecting the cells from the damage caused by free radicals and helps combat skin ageing. It acts at several levels and is an effective hypopigmenting agent. Resveratrol is not only a direct inhibitor of tyrosinase, but also an indirect inhibitor thereof, determining a reduction in hyperpigmentation, typical of the periocular area. Furthermore, it can influence the keratinocytes, which regulate the function of the melanocytes. Resveratrol in fact regulates the inflammatory process of the keratinocytes and protects them from oxidative damage. Furthermore, it appears to be effective also against UV-induced damage. Restoration of the first line of defence mechanism in a chronic animal model exposed to UV rays has shown that Resveratrol can be developed as an innovative cosmetic product for significant improvement and repair of photo-damaged skin. According to an aspect of the present invention, Resveratrol is present in a concentration between 0.01% and 1% by weight with respect to the total weight of the composition, preferably equal to 0.1 %. According to another aspect of the invention, Resveratrol is extracted from the roots, leaves and stem of *Polygonum cuspidatum,* through a process that entails the following steps: i) extraction with 95% ethanol; ii) dissolution in water and filtering; iii) hydrolysis; iv) liquid-liquid extraction; v) elution.

The Acetylhexapeptide-8 is a molecule with botox-like action, having activity similar to that exercised by the botulinum toxin when it is injected into the expression lines caused by continuous and repeated contraction of the facial muscles.

The structure of the Acetylhexapeptide-8 is the following 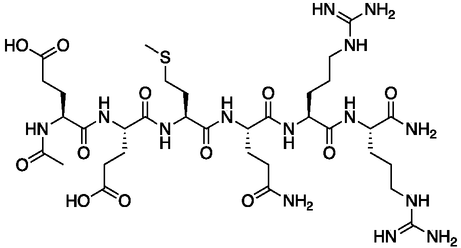 and it is able to induce decontraction of the mimetic muscles, with consequent temporary relaxation of the wrinkles caused by them. It performs its action by topical application, providing a safe and non-invasive cosmetic alternative to injections of type A botulinum toxin.

Once it has penetrated into the skin, thanks to its small dimensions, it acts by inhibiting release of the endogenous messengers of muscle contraction. According to an aspect of the present invention, the Acetylhexapeptide-8 is present in a concentration between 0.03% and 3% by weight with respect to the total weight of the composition, preferably equal to 0.3%.

Mullein (*Verbascum thapsus)* is a biennial plant found throughout the Mediterranean area, with flowers having corolla with five yellow petals. More than 200 bioactive constituents (phenylethanoids, flavonoids, neolignan glycosides, phenolic acids, iridoids, saponins and polysaccharides) have already been isolated from various species of Mullein, and today we are ever closer to knowing its anti-ageing and anti-inflammatory properties. Firstly there are the iridoids, molecules typical of this species, that have the ability to re-emit, at greater wavelength and therefore at lower energy, the electromagnetic radiations received, and absorb radiations in the ultraviolet part of the spectrum and emit them in the visible part. Recent studies have highlighted the luminescent properties of extract of mullein flowers: these flowers in fact have the ability to absorb UV luminous energy and emit it in the form of visible light, and use of the molecule has shown illuminating properties at skin level. According to an aspect of the present invention, mullein extract is present in a concentration between 0.1% and 10% by weight with respect to the total weight of the composition, preferably equal to 1%. According to a further aspect of the present invention, the mullein extract is obtained from the leaves and flowers which are dried when in full bloom.

The combination of these active substances present in the composition in question results in synergic effects. For example, Vitamin K and Resveratrol have a multitarget action on periocular depigmentation. Vitamin K is able to determine a reduction in depigmentation by acting on the vascular component, therefore strengthening the capillary wall, and it avoids the deposit of haemosiderin and haemoglobin degradation products. However, periocular pigmentation is linked not only to the local accumulation of haemoglobin oxidised products, since the face is the most photo-exposed part of the body, periocular pigmentation is produced also by a dysregulation of melanogenesis, with localized accumulations of melanin that cannot be controlled solely by Vitamin K. This is where Resveratrol intervenes, able to act on tyrosine, the target enzyme in the production of melanin, which reduces the local accumulation of melanin and modulates the production thereof. The association of Vitamin K and Resveratrol demonstrates the synergy of the combination of the two molecules: Vitamin K reduces the depigmentation linked to capillary weakness and the Resveratrol reduces the depigmentation linked to UV-induced damage, acting together to solve localized hyperpigmentation.

The association of Vitamin K, Resveratrol and mullein extract also results in an important synergy. Vitamin K in fact acts by reducing the depigmentation induced by the accumulation of haemoglobin degradation products and, in synergy with the melanogenesis suppression activity of the Resveratrol, provides an effective combined action. However, it is fundamental to insert molecules that act upstream of both the Resveratrol and the Vitamin K, blocking and reflecting the ultraviolet rays, such as the iridoids present in mullein extract. The rationale for the combined use of the iridoids, molecules able to reflect the ultraviolet light, lies not only in giving the skin radiance in the area of use, but also in screening the cells from the action of the ultraviolet rays and this takes place in synergy with the other two active substances since it acts upstream of the melanogenesis biosynthetic pathway, directly blocking the solar radiation. The effect of physical blocking and refraction, with a reduction in UV ray incidence, reduces the melanogenesis, which is suppressed and modulated by the Resveratrol. Therefore, the combination of the iridoids, which refract the sun's rays, with the Resveratrol, which suppresses the melanogenesis, and the Vitamin K, increases the depigmenting effect by acting on different targets of the same metabolic pathway.

Since chronic photoexposure and protracted contraction of the muscles in the periocular area cause the onset of surface and medial wrinkles, it is important to act on the environmental causes, namely the ultraviolet rays, and, simultaneously, attenuate the exogenous factors, i.e. the contraction of the mimetic muscles. It is therefore particularly interesting to associate molecules that act synergically on these fronts. Acetylhexapeptide-8 is a molecule able to induce decontraction of the mimetic muscles, with consequent temporary relaxation of the wrinkles caused by them, but, as said previously, the onset of periocular wrinkles is also caused by exposure to ultraviolet rays. Therefore, its combination with molecules that protect against solar radiation like Resveratrol and the iridoids results in a synergic action against the two main etiological factors underlying wrinkles. In parallel, the Resveratrol, in addition to inhibiting damage induced by UV rays, demonstrated myopreservative properties of the neuromuscular plaque, namely it modulates the release of neurotransmitters, including acetylcholine, favouring muscular trophism and regulating contraction. Therefore, Resveratrol in combination with the Acetylhexapeptide-8 guarantees a reduction in contraction of the periocular muscles, preserving their structure and modulating their activity.

Dark circles are caused by damage to the microcirculation, blood extravasation and the accumulation of liquids. As seen previously, it is possible to act on the pigmentation and on the muscle contraction of the eye contour, but combined treatment at several levels is recommended. Bromelain strengthens the capillary microcirculation and promotes edema reabsorption at periocular level, acting both upstream and downstream of the liquid accumulation process. However, damage to the microcirculation is complex, involving various factors, including the formation of reactive oxygen species (ROS), endothelial damage, blood extravasation and activation of the coagulation cascade. The importance of the synergy between Bromelain, Vitamin K and Resveratrol is fundamental as the Resveratrol inhibits the formation of ROS, acting as an antioxidant, the Vitamin K regulates the local coagulation, preventing the accumulation of haemoglobin degradation compounds, and the Bromelain reduces the local edema, activating enzymes able to break down any products accumulated from the blood extravasation. The synergic action of Bromelain and Resveratrol leads to inhibition of the formation of proinflammatory interleukins underlying the formation of the ROS. According to an embodiment of the invention in question, the composition can further comprise escin, present in a concentration between 0.001% and 0.1% by weight with respect to the total weight of the composition, preferably equal to 0.01%.

Escin, the active ingredient of horse chestnut extract, shows anti-inflammatory, anti-edemigenous and capillarotropic activity. It reduces the action of the elastase and the hyaluronidase, enzymes which in the case of phlogosis break down the internal structure of the vessels, weakening the endothelium of the capillaries and leading to the formation of edema. The action on these proinflammatory hydrolases guarantees the physiological restoration of resistance and permeability of the vessels (anti-edemigenous effect).

According to another embodiment of the invention in question, the composition can further comprise caffeine, present in a concentration between 0.005% and 0.5% by weight with respect to the total weight of the composition, preferably equal to 0.05%.

The results show that caffeine protects the skin from senescence induced by oxidative stress, activating autophagy via a series of sequential events starting from inhibition of its adenosine cell receptor A2a (A2AR) through to increase in the proteic level of Sirtuin 3 (SIRT3) and activation of the Adenosine MonoPhosphate-activated protein Kinase (AMPK), all molecules involved in the inflammation containment processes and in modulation of the cell energy activity. Caffeine has anti-edemigenous properties, namely the capacity to reduce the presence of excess liquids in the tissues, since it stimulates drainage of the liquids (it is particularly suitable for the treatment of swollen eyes). The use of caffeine is particularly suitable also for protecting the eye contour area, in particular the capillaries, from UV rays, having shown effectiveness in preventing UV-induced damage.

The authors of the present invention have also specified that the composition, in any embodiment discussed here, can further comprise centella asiatica, in a concentration between 0.25% and 25% by weight with respect to the total weight of the composition, preferably equal to 2.5%. Centella asiatica (C. asiatica) is a medicinal plant from South Asia. It has been shown that extracts of C. asiatica containing various pentacyclic triterpenes stimulate the synthesis of collagen in the deepest layers of the epidermis, countering the loss of elasticity and the formation of wrinkles. Furthermore, extracts of C. asiatica could regulate stress-induced premature senescence (SIPS) by preventing repression of DNA replication and genic expression correlated with mitosis.

According to a further embodiment of the invention in question, the composition can also comprise vitamin E, in a concentration between 0.07% and 7% by weight with respect to the total weight of the composition, preferably equal to 0.7%. Vitamin E is a liposoluble antioxidant and has been in use for over 50 years in dermatology. It is an important ingredient in cosmetic products as it protects the skin from various harmful effects due to solar radiation, eliminating the free radicals. Experimental studies suggest that vitamin E has antitumoral and photo-protective properties.

The present invention also concerns an embodiment of the composition also comprising hyaluronic acid, in a concentration between 0.03% and 3% by weight with respect to the total weight of the composition, preferably equal to 0.3%. Hyaluronic acid is a simple linear polymer in which a disaccharide is repeated thousands of times, thus creating an enormous hydrophilic molecule that confers a large volume of hydration and contributes to the turgor and suppleness of the skin. An in-depth analysis of the literature has revealed that hyaluronic acid-based formulations have considerable anti-ageing and filling properties. Its effect is an increased density of the soft tissues, improved skin hydration, in addition to stimulating collagen and elastin and restoring face volume. According to another embodiment of the invention in question, the composition can further comprise vitamin C, in a concentration between 0.01% and 1% by weight with respect to the total weight of the composition, preferably equal to 0.1%. Vitamin C (or ascorbic acid) is the vitamin "par excellence" as it takes part in numerous biological processes and physiological mechanisms that are "protective" for the organism. The skin normally contains high concentrations of vitamin C as it supports important and well-known functions, stimulating the synthesis of collagen and assisting the antioxidant protection against photo-damage induced by UV rays. In the cosmetic field, vitamin C is known above all for its powerful antioxidant activity. This is expressed through the scavenger effect, namely it has an effect against the reactive oxygen species, and in the regeneration of α-tocopherol (vitamin E), starting from its oxidised form. On the one hand, therefore, vitamin C is able to provide direct anti-radical protection (it reduces the chemical reactivity and, consequently, the toxicity of the free radicals), and on the other, it is able to amplify the benefits ensured by other antioxidant substances (including vitamin E) and takes part in the metabolic processes that lead to their physiological regeneration. Furthermore, vitamin C is fundamental for maintaining the integrity of the collagen fibres also of the blood vessels, fortifying them, and reducing the deposition of pigment in the periocular area.

According to an embodiment of the composition subject of the present invention, the composition can further comprise shea butter, which has a concentration between 0.2% and 20% by weight with respect to the total weight of the composition, preferably equal to 2%. Shea butter is an edible substance extracted from the seeds, nuts, of the shea tree or *Vitellaria paradoxa.* It is composed of stearic acid and oleic acid, which together represent 85% of the total fatty acids. It contains sterols, tocols and terpenes; of these, the most significant, over 50% of the total, is α-amyrin. Due to its composition, shea butter provides effective skin hydration, restoring the epidermal barrier, and aiding protection against external physical aggression.

According to a further embodiment of the invention in question, the composition can also comprise oak extract in a concentration between 0.05% and 5% by weight with respect to the total weight of the composition, preferably equal to 0.5%. Oak extract provides mainly condensed tannins (catechins, ellagitannins, proanthocyanidins), in a percentage estimated between 8 and 20%, but also resins, pectins and flavonoids. All these molecules perform an antioxidant function and reduce UV-induced damage, favouring the local microcirculation and reducing inflammation.

Other embodiments of the composition subject of the present invention can comprise Vitamin K, Bromelain, Resveratrol, Acetylhexapeptide-8, mullein extract and one or more of the following active substances: escin, caffeine, centella asiatica, Vitamin E, Vitamin C, hyaluronic acid, shea butter and/or oak extract.

In a preferred embodiment, the composition, according to any one of the embodiments described, comprises Vitamin K, in a concentration equal to 0.01% by weight with respect to the total weight of the composition, Bromelain, in a concentration of 0.1% by weight with respect to the total weight of the composition, Resveratrol, in a concentration of 0.1% by weight with respect to the total weight of the composition, Acetylhexapeptide-8, in a concentration of 0.3% by weight with respect to the total weight of the composition, mullein extract, in a concentration of 1% by weight with respect to the total weight of the composition, escin, in a concentration of 0.01% by weight with respect to the total weight of the composition, caffeine, in a concentration of 0.05% by weight with respect to the total weight of the composition, centella asiatica, in a concentration of 2.5% by weight with respect to the total weight of the composition, Vitamin E, in a concentration of 0.7% by weight with respect to the total weight of the composition, Vitamin C, in a concentration of 0.1% by weight with respect to the total weight of the composition, hyaluronic acid, in a concentration of 0.3% by weight with respect to the total weight of the composition, shea butter, in a concentration of 2% by weight with respect to the total weight of the composition, and oak extract, in a concentration of 0.5% by weight with respect to the total weight of the composition.

According to any one of the embodiments described here, the composition is preferably for topical use and can be formulated as one of the following forms: cream, ointment, paste, hydrophilic gel, hydrophobic gel, foam, emulsion, suspension or liquid. According to the type of formulation, the composition can further comprise one or more excipients and/or carriers. For example, if the formulation is a lipophilic ointment, the excipients to be added could be one or more out of solid, semi-solid and/or liquid paraffins, vegetable oils, waxes, liquid silicones, which generally have occlusive or protective properties. Said composition formulated as an ointment could also comprise surfactants, such as sorbitan esters, lanolin alcohols, fatty alcohols, fatty acid sulphates, fatty acid esters and/or polysorbates in order to obtain an ointment that emulsifies in water.

If, on the other hand, the present invention is formulated as a hydrophilic ointment, the composition could contain mixtures of polyethylene glycols.

Another possible embodiment of the present invention is the composition formulated as a cream. Since cream is a multi-phase preparation, consisting of a lipophilic phase and a hydrophilic phase, the addition of emulsifiers is fundamental. If the composition is formulated as a hydrophobic cream, it can also contain one or more of the following emulsifiers: wool alcohols, sorbitan esters and monoglycerides. If, on the other hand, it is formulated as a hydrophilic cream, one or more emulsifiers could be comprised in the composition will be one or more of sodium soaps, polysorbates and/or fatty alcohol sulphates.

According to an embodiment of the composition in question, it can be formulated also as a hydrophilic gel, also containing one or more of the following excipients: sodium alginate, agar-agar, gums, gelatin, starch, derivatives of cellulose and/or carboxyvinyl polymers.

Another embodiment is the composition formulated as hydrophobic gel, comprising one or more of the following excipients: micronized silica, hydrogenated castor oil, stearyl ammonium hectorite, zinc stearates, calcium stearates and/or aluminium stearates.

All the embodiments described here could also comprise moisturizing substances such as collagen, elastin, various protein hydrolysates, nucleic acid hydrolysates, NMF and/or natural moisturizing factor, absorption promoters, like DMSO, fatty acids, urea azone^{™} and/or menthol, fragrances, essences and/or preservatives.

Any embodiment described here can be produced through mixing techniques for mixing the active substances, known to persons skilled in the art.

The use of the composition, in any one of the embodiments described, has the purpose of preventing, reducing and/or eliminating skin blemishes in the periocular area. The composition or any one embodiment thereof can be applied on the facial skin, in particular in the eye contour area, with the fingers or with a make-up sponge, preferably after thorough face cleansing. Once applied, the composition in question is massaged into the area of interest until it is completely absorbed. The use of the composition or any one embodiment thereof entails the use of a small quantity at least twice a day, preferably in the morning and in the evening.

It is possible to apply the composition or any one embodiment thereof before or in combination with other face care or make-up products.

Preferably the composition in question will be kept at ambient temperature, in a cool dry place, well away from sources of light and heat, which could cause deterioration of the product.

As mentioned, the subject of this invention is also a cosmetic product comprising or consisting of the composition according to any one of the embodiments described here in the form of serum, face mask, make-up remover wipe, cleansing wipe, moisturizing cream, lifting cream, moisturizing gel, make-up removers, exfoliants, toners, micellar water or make-up product.

The composition or the cosmetic product comprising or consisting of the composition, according to any one embodiment described here, is applied in order to prevent, reduce and/or eliminate blemishes such as bags under the eyes, dark circles, periocular wrinkles and/or hyperpigmentation. Dark circles are a fairly widespread imperfection, spoiling facial beauty and expression. These dark bluish marks that appear below the eye indicate congenital capillary fragility. Dark circles are therefore not simply a sign of ageing, but an imperfection that also affects younger people. In addition to capillary fragility, the appearance of dark circles is linked to a long list of predisposing factors, such as individual hereditariness. In fact, it is believed that dark circles can appear in an individual based on a genetic predisposition, in addition to being due to incorrect habits and lifestyles characterized by excessive psychophysical stress.

The thin layer of skin below the orbital cavity is rich in very fine blood vessels. When these microscopic capillaries break, they release small drops of blood which tend to expand in the tissues where they remain for a long time. This stagnation gives rise to the characteristic dark rings that distinguish dark circles from other blemishes, such as bags or wrinkles. The rupture of capillaries can be favoured not only by their innate fragility but also by incorrect behavioural habits. In fact, dark circles can be accentuated by thermal stress (sudden differences in temperature, direct exposure to sunlight or face washing with boiling water) and/or mechanical stress (continuous rubbing of the eyes or excessive contraction of the face capillaries mediated by catecholamines, hormones secreted in response to high levels of psychophysical stress). At the same time, insufficient sleep favours the stagnation of blood around the orbits, facilitating the microhaemhorrages responsible for dark circles. Lastly, in some cases, dark circles can be caused by an accumulation of the pigment melanin in the skin below the eyes.

Bags are also blemishes typical of the eye contour: typical puffiness below the eyes is more marked in given circumstances (when waking up in the morning, in conditions of great stress or intense reading, after a very salty meal, etc.) and is very evident in older people. As we know, the eyelids are formed of folds of skin tissue with extraordinary elasticity, making them very extensible. However, this characteristic means that the eyelids can be easily "attacked" by phenomena of premature skin ageing. Over time, the connective tissue that forms the subcutaneous cell layer of the eyelids tends to lose its extensibility, gradually relaxing, until it gives rise - due to the contribution of local retention - to the phenomenon of bags, widely considered responsible for a "dull" look.

In addition to dark circles and bags, wrinkles are another imperfection typical of the eye contour. The 20 muscles in the eye contour area ensure our facial expressions with over 10,000 daily movements. The same muscles, however, also play a decisive role in the formation of wrinkles, crow's feet and other ageing lines.

The term hyperpigmentation is used in medicine to indicate skin areas that become darker than the surrounding areas. Hyperpigmentation includes age spots, melasma and post-inflammatory hyperpigmentation. Hyperpigmentation of the skin is a phenomenon that can be triggered by multiple factors, including age. Although this imperfection is often associated with the ageing process, it has been shown that the spots can appear also during adolescence.

The subject of the present invention is the use of the composition, according to any one of the embodiments described here, to prevent, reduce and/or eliminate periocular skin blemishes.

Capillary fragility is a very common problem, especially among women. There are numerous causes, both benign and pathological. Genetic predispositions, nutritional deficiencies (Vitamin C, Vitamin P), temporary hormonal alterations (pregnancy, pre-menstrual phase) and incorrect behavioural habits (excessive exposure to the sun, lack of physical activity, excess weight/obesity, stress) often coexist. Capillary fragility manifests itself with the appearance of pinpoint haemorrhagic spots, which can be more or less extensive (petechiae, ecchymosis and haematoma), especially on the face and legs. When this situation is particularly evident, it denotes a condition called purpura, in which the haemorrhagic manifestations tend to appear spontaneously or after slight traumas.

Swelling of the eyelid occurs due to the presence of excess liquids in the connective tissues of the periocular region (around the eye). This accumulation of liquid is defined palpebral edema. The swelling of the eyelids can be monolateral or bilateral, namely it can affect only one or both of the eyelids. Furthermore, it can be asymptomatic or accompanied by itching or pain.

Palpebral edema has numerous causes. It can depend on the conditions directly affecting the eyelid but it can also originate from pathologies affecting the structures in and around the ocular orbit. In other cases, eyelid swelling is caused by systemic diseases responsible for generalized edema.

Herewith disclosed is also the composition, according to any one of the embodiments considered, for use in a method for the treatment of capillary fragility and/or edema, entailing at least one application of said composition at least twice a day, preferably one in the morning and one in the evening, in the periocular area.

### EXAMPLES

### Example 1. Composition example

Emulsion comprising: Escin (001%); Resveratrol (0.1%); Hyaluronic acid (0.3%); Acetylhexapeptide-8 (0.3%); Vitamin K (0.01%); Vitamin C (0.1%); Vitamin E (0.7%); Caffeine (0.05%); Bromelain (0.1%); Shea butter (2%); Centella Asiatica extract (25%); oak extract (0.5%); mullein extract (1%).
Smell: characteristic
Colour: beige
pH: 5.5-6.5
Density: 0.95- 0.98 g/ml
Solubility in water: non-soluble

### Example 2. Comparative effectiveness studies

### Materials and Methods

In order to test the effectiveness of the composition subject of the invention with respect to the single active ingredients that compose it in the cosmetic treatment of periocular skin, 15 subjects were recruited at the dermatological clinic of Policlinico di Roma Tor Vergata. The subjects recruited were divided into 5 arms (3 subjects per arm) in order to evaluate the effect of the complete composition and the single active ingredients after 21 days of topical application, twice a day. In each arm, based on a particular topical formulation, 9 parameters were evaluated: skin colour, pigmentation, fine lines, wrinkles, pores, redness, texture, volumes and furrows. The parameters were evaluated using an *in vivo* skin imaging method by means of Miravex (Limited features Antera 3D, Dublin).

The following compositions were studied:
(1) Composition in a form for topical use according to an embodiment of the present invention, comprising: Resveratrol (0.1 %); Acetylhexapeptide-8 (0.3%); Vitamin K (0.01 %); Bromelain (0.1 %); and mullein extract (1%).
(2) Base cream/carrier + Acetylhexapeptide-8 (0.3%).
(3) Base cream/carrier + Vitamin K (0.01%).
(4) Base cream/carrier + mullein extract (1%).
(5) Base cream/carrier + Bromelain (0.1%).

### Ad interim results

As can be seen from the results reported in Figures 1-11 and in the following Table 1, the composition (1) according to an embodiment of the present invention showed an improvement in all 9 parameters examined (i.e. skin colour, pigmentation, fine lines, wrinkles, pores, redness, texture, volumes). The results obtained show that the "skin texture" parameter is improved only following administration of the above-mentioned composition (1) and not following application of the single active ingredients (compositions (2)-(5)), therefore highlighting a synergy of the active ingredients present in the composition of the invention not predictable based on the results obtained for the single ingredients administered individually.

Composition (2) - base cream/carrier + Acetylhexapeptide-8 (0.3%): the ingredient examined showed improvement in the "colour", "pores", "fine lines" and "wrinkles" parameters, without significant alteration of the "pigmentation" and "redness" parameters.

Composition (3) - base cream/carrier + Vitamin K (0.01%): the ingredient examined showed improvement in the "redness" parameter, without significant alteration of the "pigmentation", "colour", "fine lines" and "wrinkles" parameters.

Composition (4) - base cream/carrier + mullein extract (1%): the ingredient examined showed improvement in the "pigmentation" and "pores" parameters, without significant alteration in the "colour", "redness", "fine lines" and "wrinkles" parameters.

**Table 1**

| Composition | **skin colour** | **pigmentation** | **fine lines** | **wrinkles** | **pores** | **redness** | **furrows** | **texture** | **volumes** |
|---|---|---|---|---|---|---|---|---|---|
| **Resveratrol (0.1%); Acetylhexapeptide-8 (0.3%); Vitamin K (0.01%); Bromelain (0.1%); mullein extract (1%)** | X | X | X | X | X | X | X | X | X |
| **base cream/carrier + Vitamin K (0.01%)** | X | | | | | X | | | |
| **base cream/carrier + Bromelain (0.1%)** | | | | | | | | | |
| **base cream/carrier + Acetylhexapeptide-8 (0.3%)** | X | | X | X | X | X | | | |
| **base cream/carrier + mullein extract (1 %)** | | X | | | X | | | | |

Escin (0.01%); Resveratrol (0.1%); Hyaluronic acid (0.3%); Acetylhexapeptide-8 (0.3%); Vitamin K (0.01%); Vitamin C (0.1%); Vitamin E (0.7%); Caffeine (0.05%); Bromelain (0.1%); Shea butter (2%); Centella Asiatica extract (2.5%); Oak extract (0.5%); Mullein extract (1%).

## Claims

1. Composition comprising Vitamin K, Bromelain, Resveratrol, Acetylhexapeptide-8 and mullein extract.

2. The composition according to claim 1, further comprising one or more active ingredients selected from escin, caffeine, centella asiatica, Vitamin E, Vitamin C, hyaluronic acid, Shea butter and/or oak extract.

3. The composition according to any one of claims 1 or 2, wherein vitamin K is present in a concentration between 0.001% and 0.1 % and/or wherein bromelain is present in a concentration of 0.01% and 1% and/or wherein resveratrol is present in a concentration between 0.01% and 1% and/or wherein acetylhexapeptide-8 is present in a concentration between 0.03% and 3% and/or in wherein the mullein extract is present in a concentration between 0.1% and 10%, wherein said percentages are percentages by weight with respect to the total weight of the composition.

4. The composition according to any one of claims 2-3, wherein the escin is present in a concentration between 0.001% and 0.1% and/or caffeine is present in a concentration between 0.005% and 0.5% and/or centella asiatica is present in a concentration between 0.25% and 25% and/or vitamin E is present in a concentration between 0.07% and 7% and/or hyaluronic acid is present in a concentration between 0.03% and 3% and/or vitamin C is present in a concentration between 0.01% and 1% and/or shea butter is present in a concentration between 0.2 % and 20% and/or the oak extract is present in a concentration between 0.05% and 5%, wherein said percentages are percentages by weight with respect to the total weight of the composition.

5. The composition according to any one of claims 1 to 4, further comprising one or more excipients and/or carriers.

6. The composition according to any one of claims 1 to 5 for topical use.

7. The composition according to any one of claims 1 to 6 in a form selected from cream, ointment, paste, hydrophilic gel, hydrophobic gel, foam, emulsion, suspension or liquid.

8. Cosmetic product comprising or consisting of the composition according to any one of claims 1 to 7, in the form of a serum, face mask, transdermal patch, make-up remover wipe, cleansing wipe, moisturizing cream, lifting cream, moisturizing gel, make-up removers, exfoliators, tonics, micellar water, make-up product.

9. Cosmetic use of the composition according to any one of claims 1 to 7 to prevent, reduce and/or eliminate blemishes of the periocular skin.

10. Use according to claim 9, wherein said blemishes include bags under the eyes, dark circles, wrinkles, and/or periocular hyperpigmentation.

11. Use according to any of claims 9 to 10, wherein said composition is applied to the eye contour area twice a day.

## Patentansprüche

1. Zusammensetzung, umfassend Vitamin K, Bromelain, Resveratrol, Acetylhexapeptid-8 und Königskerzenextrakt.

2. Zusammensetzung nach Anspruch 1, ferner umfassend einen oder mehrere Wirkstoffe, die ausgewählt sind aus Aescin, Koffein, Centella asiatica, Vitamin E, Vitamin C, Hyaluronsäure, Sheabutter und/oder Eichenextrakt.

3. Zusammensetzung nach einem der Ansprüche 1 oder 2, wobei Vitamin K in einer Konzentration zwischen 0,001 % und 0,1 % vorliegt und/oder wobei Bromelain in einer Konzentration von 0,01 % und 1 % vorliegt und/oder wobei Resveratrol in einer Konzentration zwischen 0,01 % und 1 % vorliegt und/oder wobei Acetylhexapeptid-8 in einer Konzentration zwischen 0,03 % und 3 % vorliegt und/oder wobei der Königskerzenextrakt in einer Konzentration zwischen 0,1 % und 10 % vorliegt, wobei die Prozente Gewichtsprozente in Bezug auf das Gesamtgewicht der Zusammensetzung sind.

4. Zusammensetzung nach einem der Ansprüche 2 bis 3, wobei das Aescin in einer Konzentration zwischen 0,001 % und 0,1 % vorliegt und/oder Koffein in einer Konzentration zwischen 0,005 % und 0,5 % vorliegt und/oder Centella asiatica in einer Konzentration zwischen 0,25 % und 25 % vorliegt und/oder Vitamin E in einer Konzentration zwischen 0,07 % und 7 % vorliegt und/oder Hyaluronsäure in einer Konzentration zwischen 0,03 % und 3 % vorliegt und/oder Vitamin C in einer Konzentration zwischen 0,01 % und 1 % vorliegt und/oder Sheabutter in einer Konzentration zwischen 0,2 % und 20 % vorliegt und/oder der Eichenextrakt in einer Konzentration zwischen 0,05 % und 5 % vorliegt, wobei die Prozente Gewichtsprozente in Bezug auf das Gesamtgewicht der Zusammensetzung sind.

5. Zusammensetzung nach einem der Ansprüche 1 bis 4, ferner umfassend einen oder mehrere Hilfsstoffe und/oder Träger.

6. Zusammensetzung nach einem der Ansprüche 1 bis 5 für eine topische Verwendung.

7. Zusammensetzung nach einem der Ansprüche 1 bis 6 in einer Form, die ausgewählt ist aus Creme, Salbe, Paste, hydrophilem Gel, hydrophobem Gel, Schaum, Emulsion, Suspension oder Flüssigkeit.

8. Kosmetisches Produkt, umfassend oder bestehend aus der Zusammensetzung nach einem der Ansprüche 1 bis 7, in der Form eines Serums, einer Gesichtsmaske, eines transdermalen Pflasters, eines Abschminktuchs, eines Reinigungstuchs, einer Feuchtigkeitscreme, einer Lifting-Creme, eines Feuchtigkeitsgels, von Abschminkmitteln, von Peelings, von Tonika, von Mizellenwasser, eines Make-up-Produkts.

9. Kosmetische Verwendung der Zusammensetzung nach einem der Ansprüche 1 bis 7, um Makel der periokularen Haut vorzubeugen, zu verringern und/oder zu beseitigen.

10. Verwendung nach Anspruch 9, wobei die Makel Tränensäcke, Augenringe, Falten und/oder periokulare Hyperpigmentierung einschließen.

11. Verwendung nach einem der Ansprüche 9 bis 10, wobei die Zusammensetzung zweimal täglich auf den Bereich der Augenkontur aufgetragen wird.

## Revendications

1. Composition comprenant de la vitamine K, de la bromélaïne, du resvératrol, de l'acétylhexapeptide-8 et de l'extrait de molène.

2. Composition selon la revendication 1, comprenant en outre un ou plusieurs ingrédients actifs choisis parmi l'escine, la caféine, la centella asiatica, la vitamine E, la vitamine C, l'acide hyaluronique, le beurre de karité et/ou l'extrait de chêne.

3. Composition selon l'une quelconque des revendications 1 ou 2, dans laquelle la vitamine K est présente à une concentration comprise entre 0,001 % et 0,1 % et/ou dans laquelle la bromélaïne est présente à une concentration comprise entre 0,01 % et 1 % et/ou dans laquelle le resvératrol est présent à une concentration comprise entre 0,01 % et 1 % et/ou dans laquelle l'acétylhexapeptide-8 est présent à une concentration comprise entre 0,03 % et 3 % et/ou dans laquelle l'extrait de molène est présent à une concentration comprise entre 0,1 % et 10 %, dans laquelle lesdits pourcentages sont des pourcentages en poids par rapport au poids total de la composition.

4. Composition selon l'une quelconque des revendications 2 à 3, dans laquelle l'escine est présente à une concentration comprise entre 0,001 % et 0,1 % et/ou la caféine est présente à une concentration comprise entre 0,005 % et 0,5 % et/ou la centella asiatica est présente à une concentration comprise entre 0,25 % et 25 % et/ou la vitamine E est présente à une concentration comprise entre 0,07 % et 7 % et/ou l'acide hyaluronique est présent à une concentration comprise entre 0,03 % et 3 % et/ou la vitamine C est présente à une concentration comprise entre 0,01 % et 1 % et/ou le beurre de karité est présent à une concentration comprise entre 0,2 % et 20 % et/ou l'extrait de chêne est présent à une concentration comprise entre 0,05 % et 5 %, dans lequel lesdits pourcentages sont des pourcentages en poids par rapport au poids total de la composition.

5. Composition selon l'une quelconque des revendications 1 à 4, comprenant en outre un ou plusieurs excipients et/ou supports.

6. Composition selon l'une quelconque des revendications 1 à 5 pour une utilisation topique.

7. Composition selon l'une quelconque des revendications 1 à 6 sous une forme choisie parmi crème, pommade, pâte, gel hydrophile, gel hydrophobe, mousse, émulsion, suspension ou liquide.

8. Produit cosmétique comprenant ou constitué de la composition selon l'une quelconque des revendications 1 à 7, sous la forme d'un sérum, masque facial, patch transdermique, lingette démaquillante, lingette nettoyante, crème hydratante, crème liftante, gel hydratant, démaquillants, exfoliants, toniques, eau micellaire, produit de maquillage.

9. Utilisation cosmétique de la composition selon l'une quelconque des revendications 1 à 7 pour prévenir, réduire et/ou éliminer les imperfections de la peau périoculaire.

10. Utilisation selon la revendication 9, dans laquelle lesdites imperfections comportent des poches sous les yeux, des cernes, des rides et/ou une hyperpigmentation périoculaire.

11. Utilisation selon l'une quelconque des revendications 9 à 10, dans laquelle ladite composition est appliquée sur la zone du contour des yeux deux fois par jour.
